# EUROPÄISCHE PATENTSCHRIFT

(11) **EP 3 270 910 B1**
(45) Veröffentlichungstag und Bekanntmachung des Hinweises auf die Patenterteilung: **22.05.2019**
(21) Anmeldenummer: 16710443.9
(22) Anmeldetag: 18.03.2016
(51) Int. Cl.: A61K 31/202, C07C 403/24, A23K 20/105, A23L 33/105, A23K 20/179, A23K 50/80, A23L 33/00, A61K 31/122, A61K 31/23

(54) **ASTAXANTHINZUSAMMENSETZUNGEN (IV)**
ASTAXANTHIN COMPOSITIONS (IV)
COMPOSITIONS D'ASTAXANTHINE (IV)

(30) Priorität: 19.03.2015 EP 15159833
(43) Veröffentlichungstag der Anmeldung: 24.01.2018
(73) Patentinhaber: BASF SE, 67056 Ludwigshafen am Rhein (DE)
(72) Erfinder: FELDTHUSEN JENSEN, Jesper, 55268 Nieder-Olm (DE); RAUSCH, Tamara Alexandra, 68519 Viernheim (DE); SCHOLTEN, Claudia, 68163 Mannheim (DE); SCHAEFER, Bernd, 76889 Dierbach (DE)
(74) Vertreter: BASF IP Association
(86) Internationale Anmeldenummer: PCT/EP2016/055922
(87) Internationale Veröffentlichungsnummer: WO 2016/146804

(56) Entgegenhaltungen:
- WO-A1-2014/057493
- WO-A2-2010/100232
- JP-A- H11 290 094
- US-A1- 2006 217 445
- MIAO F ET AL: "Characterization of astaxanthin esters in Haematococcus pluvialis by liquid chromatography-atmospheric pressure chemical ionization mass spectrometry", ANALYTICAL BIOCHEMISTRY, ACADEMIC PRESS INC, NEW YORK, Bd. 352, Nr. 2, 15. Mai 2006 (2006-05-15), Seiten 176-181, XP024942217, ISSN: 0003-2697, DOI: 10.1016/J.AB.2006.03.006 [gefunden am 2006-05-15]
- ZHANG SHASHA ET AL: "Preparation of (3R, 3'R)-astaxanthin monoester and (3R, 3'R)-astaxanthin from Antarctic krill(Euphausia supe", EUROPEAN FOOD RESEARCH AND TECHNOLOGY, SPRINGER VERLAG, HEIDELBERG, DE, Bd. 240, Nr. 2, 14. September 2014 (2014-09-14), Seiten 295-299, XP035426498, ISSN: 1438-2377, DOI: 10.1007/S00217-014-2327-4 [gefunden am 2014-09-14]
- SHU YANG ET AL: "Effect of Thermal Processing on Astaxanthin and Astaxanthin Esters in Pacific White Shrimp <i>Litopenaeus v</i><i>annamei</i>", JOURNAL OF OLEO SCIENCE, Bd. 64, Nr. 3, 1. Januar 2015 (2015-01-01) , Seiten 243-253, XP055274605, JP ISSN: 1345-8957, DOI: 10.5650/jos.ess14219
- H Fukami ET AL: "hemical synthesis of astaxanthin n-octanoic acid monoester and diester and evaluation of their oral absorbability.", Journal of Oleo Science, 1. Januar 2006 (2006-01-01), Seiten 653-656, XP055274519, Gefunden im Internet: URL:https://www.jstage.jst.go.jp/article/j os/55/12/55_12_653/_pdf [gefunden am 2016-05-23]

## Beschreibung

Die vorliegende Erfindung betrifft Astaxanthinzusammensetzungen zur Verwendung in Nahrungsmitteln, Nahrungsergänzungsmitteln oder Futtermitteln oder als Medikament.

Astaxanthin (3,3'-Dihydroxy-ß,ß-carotin-4,4'dion) ist ein roter Carothinoid-Farbstoff aus der Gruppe der Xanthophylle, der durch nachstehende Formel beschrieben wird (dargestellt ist das all-trans-Isomer).

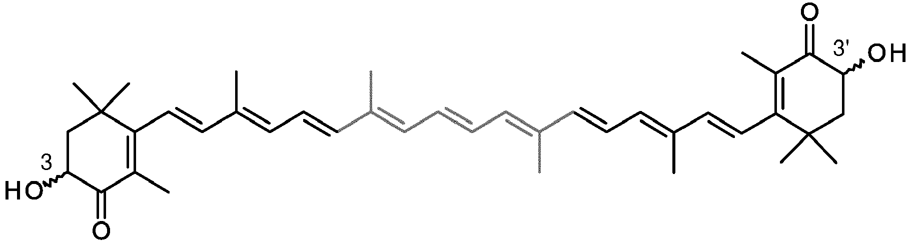

Astaxanthin, im folgenden auch AXT, weist in der 3- und 3'-Position ein Asymmetriezentrum auf und kann daher als Diastereomerengemisch der (3R,3'R)-, (3S,3'S)- und (3S,3'R)-Isomere, als Racemat des (3R,3'R)- und des (3S,3'S)-Isomers oder in Form der reinen Isomere vorliegen. Synthetisches AXT ist häufig eine Mischung der Diastereomeren (3S,3'S), (3R,3'S) und (3R,3'R). Aus natürlichen Quellen gewonnenes AXT kann, in Abhängigkeit von der jeweiligen natürlichen Quelle, in nahezu reiner (3S, 3'S) oder (3R, 3'R) Form vorliegen. Ebenso ist enantiomerenreines Astaxanthin durch Totalsynthese zugänglich.

Astaxanthin wird überwiegend synthetisch hergestellt - siehe hierzu G. Britton, S. Liaanen-Jensen, H. Pfander (Herausgeber), Carotenoids, Vol. 2, Birkhäuser Verlag, Basel, 1996, insbesondere S. 11, S. 267 ff. und S. 281 ff. und dort zitierte Literatur; B. Schäfer, Naturstoffe der chemischen Industrie, Akademischer Verlag, Heidelberg, 2007, S. 427 ff. und dort zitierte Literatur; EP 1197483, EP 1285912.

Neben der synthetischen Astaxanthin-Herstellung kann Astaxanthin in industriellem Maßstab auch aus der Blutregenalge *(Haematococcus pluvialis)* gewonnen werden (E.A. Johnson, G.H. An, Crit. Rev. Biotechnol. 1991, 11: 297-326). Man erhält hierbei ein komplexes Gemisch aus Mono- und Difettsäureestern des Astaxanthins - siehe hierzu auch D. Breithaupt, J. Agric. Food. Chem. 2004, 52: 3870-3875.

Astaxanthin ist als Futtermittelzusatzstoff zugelassen und wird in erster Linie als Futtermittelbestandteil für verschiedene Tiere verwendet, insbesondere für Lachse und Forellen. So hat AXT eine vitaminartige Wirkung wirkt sich dadurch positiv auf die Fruchtbarkeit und die Immunabwehr der Fische in Zuchtanlagen aus und führt zu einer ansprechenden Pigmentierung des Fleischs. Astaxanthin wird auch als Additiv für Nahrungsergänzungsmittel, als kosmetisches Additiv mit antioxidativen Eigenschaften oder als Nahrungsmittelfarbstoff verwendet. AXT kann die Haut vor dem durch UV-Strahlen ausgelösten Stress schützen und wirkt in dieser Funktion wesentlich stärker als Vitamin E. AXT ergänzt die Schutzwirkung von Sonnenschutzmitteln und ist nicht abwaschbar.

In jüngerer Zeit wurde berichtet, dass AXT grundsätzlich zur Behandlung einer Reihe von verschiedenen Gesundheitsstörungen geeignet ist - siehe z. B. J.H. Weisburger, Am. J. Clin. Nutr. 1991, 53: 226S - 237S; M. Guerin, Trends in Biotechnology, 2003, 21: 210-216; Ghazi Hussein et al., J. Nat. Prod., 2006, 69: 443-449, Astaxanthin and Human Health - Literature Survey (Algatechnologies): http://www.astapure-algatech.com/Data/Sub2_1/Files/algatechnologies.pdf jeweils mit weiteren Nachweisen.

So lassen in-vivo Untersuchungen an Tiermodellen vermuten, dass AXT den Blutzuckerspiegel senkt und verschiedene Parameter des metabolischen Syndroms verbessert (K. Uchiyama, Redox. Rep. 2000, 7(5): 290-3, G. Hussein et al., J. Pharm. Sci. 2007, 100(Suppl. 1):176p, M. Ikeuchi et al. Biosci. Biotechnol. Biochem. 2007, 71:893-9; Naito, Y., K. Uchiyama, et al. Biofactors 2004, 20(1): 49-59).

In Bluthochdruckmodellen führt AXT zu einer Steigerung des Blutflusses und des vaskulären Tonus - siehe z. B. H. Yanai et. al. (2008) Integrated Blood Pressure Control 1:1-3.

Epidemiologische Untersuchungen legen nahe, dass Carotinoide wie Astaxanthin sich vorteilhaft auf Herzerkrankungen auswirken - D. A. Cooper et al., Nutr. Rev. 1999, 57: 210-214, J. Krappi et. al., Int. J. Nutr. Res. 2006, 77(1): 3-11. Aufgrund seiner antioxidativen Wirkung, insbesondere seiner Wirkung, die Oxidation von low-density Lipoproteinen zu inhibieren, werden Carotinoide wie Astaxanthin zur Vorbeugung gegen Arteriosklerose vorgeschlagen - siehe T. Iwamoto et al., J. Atheroscler. Thromb. 2000, 7: 216-222.

Weiterhin wurde verschiedentlich berichtet, dass die Verabreichung von AXT das Krebsrisiko verringert - siehe J.H. Weisburger, Am. J. Clin. Nutr. 1991, 53: 226S - 237S. In-vivo Untersuchungen in Mäusen zeigen, dass Astaxanthin das Tumorwachstum, insbesondere von Mammakarzinomen, Blasenkarzinomen und Lungenkarzinom verlangsamt oder gar hemmt - siehe B.P. Chew et al., Anticancer Res. 1999, 19: 1849-1853, T. Tanaka et al. Carcinogenesis 1994, 15: 15-19 und D. A. Cooper et al., Nutr. Rev. 1999, 57: 133-145. Zudem scheint AXT die Bildung des Connexin 43 zu fördern und hat daher eine chemoprotektive Wirkung gegenüber anderen Krebserkrankungen (siehe A.L. Vine et al., Nutr. Cancer 52(1) (2005), 105-113).

Astaxanthin spielt zudem eine Rolle bei der Modulierung des Immunsystems - siehe H. Jyonouchi et al. J. Nutr. 1995, 125: 1570-1573, - und wirkt entzündungshemmend J. Park, et al. Nutr. Metab. 2010, 7: 18-27, Kurashige et al. Physiol. Chem. Phys. Med. 1990, NMR22:27-38, K. Ohgami et al. Investigative Ophthalmology & Visual Sci. 2003, 44(6):2694-2701 und Y. Suzuki et al. Exp. Eye Res. 2006, 82(2): 275-281. Es wurde auch zur Behandlung von Autoimmunerkrankungen wie Morbus Crohn vorgeschlagen.

Weiterhin wird Astaxanthin zur Behandlung von Gelenkerkrankungen, insbesondere zur Verbesserung der Symptomatik von entzündlichen Gelenkerkrankungen vorgeschlagen - siehe Y. Nir, G. Spiller C. Multz C. Effect of an Astaxanthin-containing product on carpal tunnel syndrome. American College of Nutrition Annual Meeting, San Antonio, Texas, October 2002, Y. Nir, G. Spiller. Los Altos, California: Health Research and Studies Center 2002. Y. Nir, G. Spiller. J Am Coll Nutr, 2002, 21.

In Tiermodellen konnte gezeigt werden, dass die Verabreichung von AXT den Verlauf bakterieller Infektionen, insbesondere von Entzündungen des Magen-Darm-Traktes, hervorgerufen durch Infektionen mit Helicobacter pylori, vorteilhaft beeinflusst - siehe M. Bennedsen et al., Immunol. Lett. 1999, 70: 185-189.

Ferner wird Astaxanthin zur Vorbeugung von Augenerkrankungen wie Schädigung der Linse und der Retina durch UV-Strahlung, Macula-Degeneration, Katarakt und Glaukom, zur Verbesserung der männlichen Fertilität, der Gedächtnisleistung und der Aufmerksamkeit und der körperlichen Leistungsfähigkeit vorgeschlagen - K. Sawaki et al. Journal of Clinical Therapeutics & Medicines, 2002, 18: 73 - 88.

Astaxanthin kann wesentlich dazu beitragen, die Hautalterung zu verzögern und die Entstehung von Falten, Altersflecken und Sommersprossen zu reduzieren - Suganuma K et al., Jichi Medical University Journal. 2012, 35:25-33; K. Tominaga K et al., Acta Biochim Pol. 2012;59(1):43-7; E. Yamashita, Carotenoid Science. 2006;10: 91-5; A. Satoh et al. Oyo Yakuri Pharmacometrics, 2011, 80 (1/2): 7-11.

Grundsätzlich ist daher Astaxanthin für die Herstellung von Medikamenten wie auch für Futtermittel, Nahrungsmittel und Nahrungsergänzungsmittel, insbesondere solchen für die Humanernährung, von Interesse. Als problematisch erweist sich jedoch vor allem, dass Astaxanthin bei oraler Gabe nur schlecht vom menschlichen oder tierischen Organismus absorbiert wird und die erreichten Serumspiegel im Blut- oder Lymphserum nur gering sind - siehe z. B. R.M. Clark et al. Lipids 2000, 35: 803-806; J. Mercke Odeberg et al., Europ. J. Pharm. Sci. 2003, 19: 299-304, M. Osterlie et al., J. Nutr. Biochem, 2000, 11: 482-490. Ein weiteres Problem ist die geringe Löslichkeit von Astaxanthin in den für die Herstellung von Verabreichung von Nahrungsergänzungsmittel und Medikamenten geeigneten Hilfsstoffen. Zwar kann dieses Problem durch Veresterung des Astaxanthins mit Fettsäuren teilweise gelöst werden. Jedoch zeigen die Diester des Astaxanthins mit Fettsäuren bezüglich der erreichbaren Serumspiegel überraschenderweise vergleichbare Eigenschaften auf wie unverestertes Astaxanthin - siehe D.A. White et al., Aquacult. Res. 2002, 33: 343-350, Odeberg et al., Eur. J. Pharmac. Sciences 2003, 19: 299-304, Miyazawa et al., Biosci Biotechnol Biochem, 2011, 76: 1856-1858, Osterlie et al., J. Nutr. Biochem., 11, 2000: 482-490, ,Okada et al., Biosci. Biotechnol. Biochem., 2009, 73: 1928-32,. Coral-Hinostroza et al., Comparative Biochemistry and Physiology, Part C, 139, 2004: 99-110.

Es ist bekannt, dass die Absorption von Astaxanthin durch passive Diffusion in das intestinale Epithelium erfolgt - wofür eine gewisse Menge an Fett erforderlich ist und daher durch Fette in der Nahrung gefördert werden kann (siehe G.N. Coral-Hinostroza et al. Comp. Biochem. Physiol. Part C 2004, 139: 99-110 mit weiteren Nachweisen). J. Mercke Odeberg et al., Europ. J. Pharm. Sci. 2003, 19: 299-304, beschreiben, dass die orale Bioverfügbarkeit von Astaxanthin durch spezielle fettbasierte Formulierungen verbessert werden kann, wobei die hierbei Verwendeten Emulgatoren einen starken Einfluss auf die Bioverfügbarkeit haben. Der Verbesserung der oralen Bioverfügbarkeit durch Formulierungen sind jedoch Grenzen gesetzt und schränken die Einsatzmöglichkeiten des Astaxanthins ein.

Eigene Untersuchungen haben gezeigt, dass das aus der Blutregenalge (*Haematococcus pluvialis*) gewonnene "natürliche Astaxanthin", ein komplexes Astaxanthin-Fettsäureestergemisch, eine im Vergleich zu Astaxanthin oder Difettsäureestern des Astaxanthins verbesserte orale Bioverfügbarkeit aufweist. Allerdings ist die kommerzielle Verfügbarkeit des natürlichen Astaxanthins sehr beschränkt. Aufgrund der Komplexität eines solchen Gemischs ist es für pharmazeutische Anwendungen wenig attraktiv.

Es ist daher Aufgabe der vorliegenden Erfindung, die Nachteile des Standes der Technik zu überwinden und insbesondere Astaxanthinderivate oder -Zusammensetzungen bereitzustellen, die zum einen eine bessere orale Bioverfügbarkeit aufweisen und die außerdem besser zugänglich sind als das aus der Blutregenalge (*Haematococcus pluvialis*) gewonnene "natürliche Astaxanthin".

Die ältere Europäische Patentanmeldung 14184483.7 beschreibt die Herstellung von Difettsäureestern des Astaxanthins durch Veresterung des Astaxanthins mit Fettsäurechlorid in Gegenwart einer tertiären Aminbase. Setzt man anstelle des Säurechlorids die freie Säure ein, wird überwiegend der Monofettsäureester gebildet, der aus dem Reaktionsgemisch durch Abtrennung von nicht umgesetztem Astaxanthin gewonnen werden kann. Zudem ist es möglich, die Monofettsäureester durch Einsatz unterstöchiometrischer Mengen an Säurechlorid im Gemisch mit nicht umgesetztem Astaxanthin und Difettsäureester zu erhalten. Der Monoester kann aus dem Gemisch isoliert werden, beispielsweise durch Chromatographie.

Die JP 11-290094 beschreibt ein Verfahren zur Herstellung eines Astaxanthindifettsäureesters, bei dem zur Veresterung eine Lipase eingesetzt wird.

F. Miao et al., Analytical Biochemistry 2006, 352: 176-181 beschreiben die Charakterisierung von Astaxanthinestern in *Haematococcus pluvialis* durch LC(APCI)MS. Es wird die Verteilung von freiem Astaxanthin, Astaxanthinmonoestern und Astaxanthindiestern in *Haematococcus sp.* beschrieben.

Die WO 2014/057493 A1 beschreibt Astaxanthinderivate zur Prävention und Behandlung von Hitzestress. Es wird eine Zusammensetzung beschrieben, die durch Extraktion aus *Haematococcus pluvialis* erhalten wurde. Sie enthält 85-90% Monoester.

Es wurde nun überraschenderweise gefunden, dass Astaxanthinzusammensetzungen, die wenigstens 90 Gew.-%, bezogen auf das Gesamtgewicht an Astaxanthin und Astaxanthinderivaten in der Zusammensetzung, Monoester des Astaxanthins mit einer C18-1-Fettsäure enthalten, eine besonderes gute Bioverfügbarkeit aufweisen.

Gegenstand der Erfindung ist daher die Verwendung von Astaxanthinzusammensetzungen in Nahrungsmitteln, Nahrungsergänzungsmitteln oder Futtermitteln, wobei die Astaxanthinzusammensetzungen wenigstens 90 Gew.-%, bezogen auf das Gesamtgewicht an Astaxanthin und Astaxanthinderivaten in der Zusammensetzung, Monoester des Astaxanthins mit einer C18-1-Fettsäure enthalten.

Gegenstand der Erfindung sind auch die hier und im Folgenden beschriebenen Astaxanthinzusammensetzungen zur therapeutischen Verwendung als Medikament sowie als Inhaltsstoff für medizinische Zubereitungen bzw. pharmazeutische Mittel. Dementsprechend betrifft die Erfindung auch ein Medikament, das eine erfindungsgemäße Astaxanthinzusammensetzung und wenigstens ein für pharmazeutische Zwecke geeignetes Hilfsmittel bzw. Hilfsstoff enthält.

Die vorliegende Erfindung betrifft auch Additive für Nahrungsmittel, die die erfindungsgemäße Astaxanthinzusammensetzung und wenigstens einen für Nahrungsmittel geeigneten Zusatz, d. h. wenigstens einen für Nahrungsmittel geeigneten Hilfsstoff, enthalten.

Die vorliegende Erfindung betrifft weiterhin Additive für Futtermittel, die die erfindungsgemäße Astaxanthinzusammensetzung und wenigstens einen für Futtermittel geeigneten Zusatz, d. h. wenigstens einen für Futtermittel geeigneten Hilfsstoff, enthalten.

Die vorliegende Erfindung betrifft auch Nahrungsergänzungsmittel, die die erfindungsgemäße Astaxanthinzusammensetzung und wenigstens einen für Nahrungsergänzungsmittel geeigneten Zusatz, d. h. wenigstens einen für Nahrungsergänzungsmittel geeigneten Hilfsstoff, enthalten.

Es versteht sich, dass die vorgenannten Nahrungsmitteladditive, Futtermitteladditive, Nahrungsergänzungsmittel und pharmazeutische Zusammensetzung die erfindungsgemäße Astaxanthinzusammensetzung als alleinigen, Astaxanthin-haltigen Bestandteil enthalten und dass sie insbesondere kein weiteres, unverestertes Astaxanthin oder weitere Derivate des Astaxanthins enthalten.

Die Erfindung ist mit einer Reihe von Vorteilen verbunden. Zum einen weisen die erfindungsgemäßen Zusammensetzungen eine bessere orale Bioverfügbarkeit auf als unverestertes Astaxanthin oder entsprechende Diester auf und die erreichten Serumspiegel an Astaxanthin im Blutplasma sind um ein mehrfaches höher. Überraschenderweise ist die orale Bioverfügbarkeit der erfindungsgemäßen Astaxanthinzusammensetzungen sogar besser als die des "natürlichen Astaxanthins". Zudem sind die Zusammensetzungen synthetisch gut zugänglich. Nicht zuletzt aufgrund ihrer besseren oralen Bioverfügbarkeit sind die erfindungsgemäßen Zusammensetzungen auch für pharmazeutische Anwendungen besonders attraktiv.

Insbesondere sind die erfindungsgemäßen Astaxanthin-Zusammensetzungen für die Vorbeugung und Behandlung einer Vielzahl von Krankheitsbildern und gesundheitlichen Störungen geeignet, wie beispielsweise zur
- Vorbeugung und Behandlung von Gelenkerkrankungen, insbesondere entzündliche Gelenkerkrankungen wie rheumatoide Arthritis, Arthrose und des Carpaltunnelsyndroms,
- Vorbeugung und Behandlung von Augenerkrankungen wie Schädigungen der Linse oder der Retina, z. B. zur Vorbeugung von Macula degenerata, dem Katarakt oder dem Glaukom sowie dem Schutz der Linse und der Netzhaut vor Schädigungen durch UV-Strahlen,
- Vorbeugung und Behandlung von Hauterkrankungen und zur Vorbeugung und Verringerung von Hautalterung, insbesondere zur Vorbeugung und Verringerung von Hautfalten, Altersflecken und Sommersprossen,
- Vorbeugung und Behandlung von Herzerkrankungen, insbesondere zur Vorbeugung von Herzinfarkt und zur Verbesserung der Rekonvaleszenz nach einem Herzinfarkt,
- Vorbeugung und Behandlung von Diabetes Typ2 und damit verbundener Begleiterkrankungen, beispielsweise Vorbeugung und Behandlung des metabolischen Syndroms, zur Absenkung des Blutzuckerspiegels bei insulinresistenten Individuen oder zur Behandlung und Vorbeugung der diabetischen Nephropathie,
- Vorbeugung und Behandlung von Tumorerkrankungen, insbesondere Gebärmutterhalskarzinom, Blasenkarzinom, Mammakarzinom und Lungenkarzinom,
- Vorbeugung und Behandlung von Erkrankungen der Blutgefäße wie Arteriosklerose,
- Behandlung von bakteriellen Infektionen, insbesondere zur Verbesserung des Verlaufs von Erkrankungen des Magen-Darm-Traktes, die durch Infektion mit Helicobacter pylori hervorgerufen werden,
- Vorbeugung und Behandlung von entzündlichen Erkrankungen, insbesondere von Autoimmunerkrankungen wie Morbus Crohn,
- Vorbeugung und Behandlung von degenerativen Nervenstörungen, wie Parkinson,
- zur Verbesserung der männlichen Zeugungsfähigkeit,
- zur Verbesserung des Allgemeinbefindens,
- zur Verbesserung der Gedächtnisleistung und der Aufmerksamkeit,
- zur Verbesserung der körperlichen Leistungsfähigkeit,
- zur Stärkung des Immunsystems.

Eine Behandlung im erfindungsgemäßen Sinne umfasst nicht nur die Behandlung akuter oder chronischer Anzeichen, Symptome und/oder Fehlfunktionen sondern auch eine vorbeugende Behandlung (Prophylaxe) insbesondere als Rezidiv- oder Phasen-Prophylaxe. Die Behandlung kann symptomatisch, beispielsweise als Symptomsuppression ausgerichtet sein. Sie kann kurzzeitig erfolgen, mittelfristig ausgerichtet sein, oder es kann sich auch um eine Langzeitbehandlung, beispielsweise im Rahmen einer Erhaltungstherapie, handeln.

Hier und im Folgenden versteht man unter einem Futtermittel ein Mittel für die Ernährung von nicht menschlichen Lebewesen, insbesondere von nicht menschlichen Säugern aber auch von Fischen. Unter einem Futtermitteladditiv versteht man dementsprechend eine Formulierung, z. B. in Pulverform oder flüssiger Form, die bei der Herstellung eines Futtermittels in das Futtermittel eingearbeitet werden kann.

Hier und im Folgenden versteht man unter einen Nahrungsmittel ein nährstoffhaltiges Mittel für die Ernährung von Menschen, das primär der Aufnahme von Nährstoffen wie Fette, Proteine und Kohlenhydrate dient. Unter einem Nahrungsmitteladditiv versteht man dementsprechend eine Formulierung, z. B. in Pulverform oder flüssiger Form, die bei der Herstellung eines Nahrungsmittels in das Nahrungsmittel eingearbeitet werden kann.

Hier und im Folgenden versteht man unter einem Nahrungsergänzungsmittel eine zur oralen Verabreichung geeignete Formulierung, die zusätzlich zur konventionellen Nahrung vom Menschen eingenommen wird mit dem primären Ziel einer Verabreichung von Wirkstoffen, hier von Astaxanthin oder einem Derivat, um auf diese Weise eine Verbesserung des Allgemeinzustands oder des allgemeinen Wohlbefindens zu erreichen. Nahrungsergänzungsmittel sind im Grenzbereich zwischen Nahrungsmitteln und Medikamenten angesiedelt und werden im EU-Recht durch die Richtlinie 2002/46/EG geregelt.

Das hier und im Folgenden bei generischen Begriffen wie Monocarbonsäure oder Fettsäure verwendete Präfix Cₙ-Cₘ gibt einen Bereich für die Anzahl an Kohlenstoffatomen an, den ein Mitglied der durch den generischen Begriff bezeichneten Gruppe aufweisen kann.

Die erfindungsgemäßen Astaxanthinzusammensetzungen enthalten zu wenigstens 90 Gew.-% den Monoester einer C18-1-Fettsäure, insbesondere einer linearen C18-1-Fettsäure. Daneben können die erfindungsgemäßen Astaxanthinzusammensetzungen auch nicht verestertes Astaxanthin und/oder einen oder mehrere Monoester des Astaxanthins mit einer von der C18-1-Fettsäure verschiedenen Fettsäure oder einen oder mehrere Diester des Astaxanthins mit einer Fettsäure enthalten, wobei deren Anteil, d. h. die Gesamtmenge an unverestertem Astaxanthin, Diester und Monoester einer von C18-1-Fettsäuren verschiedenen Säure, erfindungsgemäß 10 Gew.-%, bezogen auf das Gesamtgewicht an Astaxanthin und Astaxanthinderivaten in der Zusammensetzung, nicht überschreitet. Die Astaxanthinzusammensetzung kann aber auch ausschließlich oder nahezu ausschließlich, d. h. zu mehr als 90 Gew.-% aus dem Monoester des Astaxanthins mit einer C18-1-Fettsäure bestehen.

Unter einer C18-1-Fettsäure versteht man eine aliphatische Monocarbonsäure, die genau eine ethylenisch ungesättigte Doppelbindung aufweist. Diese Doppelbindung kann in beliebiger Position des Kohlenwasserstoffrests der C18-1-Fettsäure angeordnet sein und ist insbesondere in der 9-Postion oder 11-Position angeordnet. Dabei hat es sich als vorteilhaft erwiesen, wenn wenigstens 40 %, insbesondere wenigstens 50 %, besonders bevorzugt wenigstens 60 % und speziell wenigstens 70 % der Doppelbindungen im C18-1-Fettsäurerest des Monoesters Z-konfiguriert sind, d. h. eine cis-Anordnung aufweisen. Die C18-1-Fettsäure ist vorzugweise unverzweigt.

Beispiele für geeignete C18-1-Fettsäuren sind Ölsäure und ihr trans-Isomer Elaidinsäure sowie Vaccensäure.

Geeignete, von C18-1-Fettsäuren verschiedene Fettsäuren umfassen gesättigte C₁₀-C₂₂-Monocarbonsäuren, einfach ungesättigte C₁₀-C₁₇- und C₁₉-C₂₂-Monocarbonsäuren und mehrfach, z. B. 2-, 3-, 4-, 5- oder 6-fach ungesättigte aliphatische C₁₀-C₂₂-Monocarbonsäuren.

Beispiele für von C18-1-Fettsäuren verschiedene aliphatische C₁₀-C₂₂-Monocarbonsäuren sind vor allem
- gesättigte aliphatische C₁₀-C₂₂-Monocarbonsäuren insbesondere geradzahlige Säuren wie Caprinsäure, Laurinsäure, Myristinsäure, Palmitinsäure, Stearinsäure, Arachinsäure und Behensäure;
- einfach ungesättigte C₁₀-C₁₇- und C₁₉-C₂₂-Monocarbonsäuren wie Myristoleinsäure, Palmitoelinsäure, C20-1-Säuren wie Icosensäure und Gadoleinsäure, C22-1-Säuren wie Cetoleinsäure und Erucasäure;
- mehrfach ungesättigte C₁₀-C₂₂-Monocarbonsäuren wie Linolsäure (C18-2-Säure), die C18-3-Säuren alpha-Linolensäure, gamma-Linolensäure, Calendulasäure, Punicinsäure, alpha-Elaeostearinsäure und beta-Elaeostearinsäure, die C20-4 Säure Arachidonsäure, die C20-5-Säuren Timnodonsäure (= Eicosapentaensäure) und Clupanodonsäure, die C22-5-Säure Docosapentaensäure und die C22-6-Säure Cervonsäure.

Bevorzugte von C18-1-Fettsäuren verschiedene Fettsäuren sind ausgewählt unter gesättigten, ein oder mehrfach ungesättigten aliphatischen, unverzweigten C₁₈-C₂₂-Monocarbonsäuren. Insbesondere ist die von C18-1-Fettsäuren verschiedene Fettsäure unter C18-0-, C18-2-, C18-3 und C18-4-Fettsäuren ausgewählt.

Die erfindungsgemäßen Astaxanthinzusammensetzungen sind solche, worin wenigstens 90 Gew.-% des Monoesters des Astaxanthins ein Monoester des Astaxanthins mit einer C18-1-Fettsäure, insbesondere der Ölsäure oder des cis/trans-Isomerengemischs der Ölsäure ist. In den Monoestern der C18-1-Fettsäure liegen vorzugweise nicht mehr als 60 %, insbesondere nicht mehr als 40 % und speziell nicht mehr als 30 % der Doppelbindungen im C18-1-Fettsäurerest in der Transform vor.

In einer Gruppe von Ausführungsformen liegen wenigstens 40 Gew.-%, insbesondere wenigstens 60 Gew.-% oder wenigstens 80 Gew.-% des Monoesters der C18-1-Fettsäure, insbesondere des Monoesters der Ölsäure als Monoester des all-E-Astaxanthins vor. Bevorzuat liegen alle in der Zusammensetzung enthaltenen Astaxanthinverbindungen, d. h. alle Monoester und gegebenenfalls enthaltene Diester und unverestertes Astaxanthin zu wenigstens 40 Gew.-%, insbesondere wenigstens 60 Gew.-% oder wenigstens 80 Gew.-% als all-E-Astaxanthinverbindungen vor.

In einer weiteren Gruppe von Ausführungsformen liegen 20 bis 90 Gew.-%, insbesondere 30 bis 80 Gew.-% oder 40 bis 70 Gew.-% des Monoesters der C18-1-Fettsäure, insbesondere des Monoesters der Ölsäure, als Monoester des all-E-Astaxanthins und 10 bis 80 Gew.-%, insbesondere 20 bis 70 Gew.-% oder 30 bis 60 Gew.-% als Monoester ein oder mehrerer Z-Isomere, z. B. als 9-Z, 13-Z oder 15-Z-Isomer des Astaxanthins oder als Monoester eines Gemischs von zweien oder drei dieser Z-Isomere des Astaxanthins vor. In dieser Ausführungsform liegen alle in der Zusammensetzung enthaltenen Astaxanthinverbindungen, d. h. alle Monoester und gegebenenfalls enthaltene Diester und unverestertes Astaxanthin zu 20 bis 90 Gew.-%, insbesondere 30 bis 80 Gew.-% oder 40 bis 70 Gew.-% als all-E-Astaxanthinverbindungen und zu 10 bis 80 Gew.-%, insbesondere 20 bis 70 Gew.-% oder 30 bis 60 Gew.-% als ein oder mehrerer Z-Isomere, z. B. als 9-Z, 13-Z oder 15-Z-Isomer oder als Monoester eines Gemischs von zweien oder drei dieser Z-Isomere vor.

In einer weiteren Gruppe von Ausführungsformen liegen wenigstens 80 Gew.-%, insbesondere wenigstens 90 Gew.-% des Monoesters der C18-1-Fettsäure, insbesondere des Monoesters der Ölsäure, als Monoester des 3S,3'S-Enantiomers des Astaxanthins vor.

In einer weiteren Gruppe von Ausführungsformen liegen wenigstens 80 Gew.-%, insbesondere wenigstens 90 Gew.-% des Monoesters der C18-1-Fettsäure, insbesondere des Monoesters der Ölsäure, als Monoester des 3R,3'R-Enantiomers des Astaxanthins vor.

In einer weiteren Gruppe von Ausführungsformen handelt es sich bei dem Monoester der C18-1-Fettsäure, insbesondere dem Monoester der Ölsäure um den Monoester eines Gemischs des 3S,3'S-Enantiomers, der 3R,3'S-meso-Form und des 3R,3'R-Enantiomers des Astaxanthins, insbesondere des statistischen Gemischs dieser Astaxanthinformen.

Gemäß einer Gruppe von Ausführungsformen der Erfindung werden die erfindungsgemäßen Astaxanthinzusammensetzungen bzw. die entsprechenden Monoester in Nahrungsmitteladditiven oder Futtermitteladditiven eingesetzt. Diese Nahrungs- bzw. Futtermitteladditive enthalten die erfindungsgemäße Astaxanthinzusammensetzung und wenigstens einen für Nahrungsmittel oder Futtermittel geeigneten Hilfsstoff bzw. Hilfsmittel. Unter einem für Nahrungsmittel bzw. Futtermittel geeigneten Hilfsstoff versteht man im Sinne der Erfindung eine für die Tier- und/oder Humanernährung zugelassene Substanz.

Geeignete Hilfsstoffe sind insbesondere solche, die eine Einarbeitung des Additivs in Nahrungsmittel erlauben, in dem sie die Astaxanthinzusammensetzung auf eine besser handhabbare Konzentration im Additiv verdünnen. Hierzu zählen vor allem Trägermaterialien, insbesondere flüssige Trägermaterialien wie Pflanzenöle und organische Lösungsmittel, aber auch feste Trägermaterialien wie Fette, Fettsäuren, Wachse, Fettalkohole und Fettsäureester von Fettalkoholen, Kohlenhydrate, Zuckeralkohole, und anorganische Füllstoffe, welche für die Herstellung von Nahrungs- bzw. Futtermitteln zugelassen sind.

Beispiele für Lösungsmittel sind:
- C₁-C₆-Alkanole, wie beispielsweise Ethanol, 1-Propanol, 2-Propanol, 1-Butanol, 2-Butanol, 2-Methyl-1-propanol, 3-Methyl-1-butanol, 1-Pentanol und deren Gemische;
- C₁-C₄-Alkylester aliphatischer C₁-C₄-Carbonsäuren, wie beispielsweise die Methyl-, Ethyl-, n-Propyl-, iso-Propyl-, n-Butyl-, 2-Butyl- oder iso-Butylester der Ameisensäure, der Essigsäure, der Propionsäure oder der Buttersäure, wie Methylacetat, Ethylacetat, n-Propylacetat, Isopropylacetat, n-Butylacetat, Isobutylacetat, Ethylformat und deren Gemische; sowie
- aliphatische, insbesondere nicht-cyclische Ketone mit 3 bis 6 C-Atomen wie Aceton, Methylethylketon, Isobutylmethylketon und deren Gemische; sowie
- Gemische der vorgenannten Lösungsmittel aus verschiedenen Klassen der vorgenannten Lösungsmittel.

Geeignete Kohlehydrate umfassen Mono-, Di-, Oligo- und Polysacharide. Beispiele für Monosaccharide und Disaccharide sind vor allem Glukose, Fruktose, Galaktose, Mannose, Maltose, Saccharose und Lactose. Geeignete Polysaccharide sind Stärke und oligomere Stärkeabbauprodukte (Dextrine) sowie Cellulosepulver.

Geeignete Zuckeralkohole sind vor allem Sorbitan und Glycerin.

Geeignete Fette und Öle können synthetischer, mineralischer, pflanzlicher oder tierischer Herkunft sein.

Beispiele für Öle sind Pflanzenöle wie Sojabohnen-Öl, Sonnenblumenöl, Maiskeimöl, Leinsamenöl, Rapsöl, Distelöl, Weizenkeimöl, Reisöl, Kokosöl, Mandelöl, Aprikosenkernöl, Palmöl, Palmkernöl, Avocadoöl, Jojobaöl, Haselnussöl, Walnussöl, Erdnussöl, Pistazienöl, Triglyceride mittelkettiger (= C₈-C₁₈) pflanzlicher Fettsäuren (sog. MCT-Öle) und PUFA-Öle (PUFA = mehrfach ungesättigte Fettsäuren (polyunsaturated fatty acids) wie Eikosapentaensäure (EPA), Docosahexaensäure (DHA) und α-Linolensäure), weiterhin semisynthetische Triglyceride, z. B. Caprylsäure/Caprinsäure-Triglyceride wie die Miglyol-Typen, weiterhin Paraffinöl, flüssige hydrierte Polyisobutene, Squalan, Squalen, weiterhin tierische Öle und Fette wie Fischöle einschließlich Makrelen-, Sprotten-, Thunfisch-, Heilbutt-, Kabeljau- und Lachsöl. Bevorzugt sind pflanzliche Öle und Öle tierischen Ursprungs, die bei 40 °C flüssig sind, insbesondere Pflanzenöle wie Sojabohnen-Öl, Sonnenblumenöl, Distelöl, Maiskeimöl, Olivenöl, Leinsamenöl, Rapsöl, Reisöl, Kokosöl, Erdnussöl, Palmöl, Palmkernöl, PUFA-Öle, MCT-Öle, weiterhin Fischöle, sowie Mischungen dieser Öle.

Fette weisen im Unterschied zu Ölen in der Regel einen Schmelzpunkt oberhalb 30 °C auf. Beispiele für Fette sind:
- gesättigte Fettsäuren mit 12 bis 30 C-Atomen (gesättigte C₁₂-C₃₀-Fettsäuren), insbesondere 14 bis 28 C-Atomen (gesättigte C₁₄-C₂₈-Fettsäuren) und speziell 16 bis 24 C-Atomen (gesättigte C₁₆-C₂₄-Fettsäuren) wie Myristinsäure, Palmitinsäure, Margarinsäure, Stearinsäure, Arachinsäure, Behensäure, Cerotinsäure, Melissinsäure und Lignocerinsäure sowie deren Gemische;
- Fettsäureester von gesättigten Fettsäuren mit 14 bis 30 C-Atomen, insbesondere 14 bis 28 C-Atomen und speziell 16 bis 24 C-Atomen wie die Ester der Palmitinsäure, Margarinsäure, Stearinsäure, Arachinsäure, Behensäure, Cerotinsäure, Melissinsäure und Lignocerinsäure sowie deren Gemische, insbesondere die Mono-, Di- und Triglyceride der vorgenannten Fettsäuren und deren Gemische (im Folgenden als Fettsäure-Glyceride bezeichnet), insbesondere der vorgenannten gesättigten Fettsäuren und speziell solcher gesättigter Fettsäuren mit 14 bis 28 C-Atomen und speziell 16 bis 28 C-Atomen, sowie Ester der vorgenannten gesättigten Fettsäuren mit vorzugsweise 14 bis 28 C-Atomen und speziell 16 bis 28 C-Atomen, mit C₁₀-C₃₀-Fettalkoholen, insbesondere mit C₁₄-C₂₈-Fettalkoholen. Die vorgenannten Ester von gesättigten Fettsäuren können bis zu 10 Gew.-%, bezogen auf die Fettsäureanteil im Ester, auch ein oder mehrfach ungesättigte Fettsäuren in veresterter Form enthalten. Insbesondere macht der Anteil ungesättigter Fettsäurebestandteile in diesen Estern weniger als 5 Gew.-% aus, bezogen auf die gesamten Fettsäurebestandteile im Ester;

Bei den Ölen und Fetten kann es sich um Raffinatöle oder Rohöle bzw. Rohfettehandeln, die noch ursprungsspezifische Verunreinigungen wie Proteine, Phosphat, (Erd)alkalimetallsalze und dergleichen in üblichen Mengen enthalten.

Als Fettalkohole kommen insbesondere gesättigte aliphatische Alkohole mit 8 bis 30 C-Atome (im Folgenden auch als C₈-C₃₀-Fettsalkohole) in Betracht, wie beispielsweise Cetylalkohol, Stearylalkohol, Nonadecanol, Arachidylalkohol, Behenylalkohol, Lignocerylalkohol, Cerylalkohol, Myricylalkohol und Melissylalkohol.

Als Wachse kommen insbesondere natürliche Wachse pflanzlichen oder tierischen Ursprungs wie Bienenwachs, Candelilawachs, Schellack-Wachs, Karitefett und Carnaubawachs, Kohlenwasserstoffwachse, wie Paraffinwachse, Ceresin, Sasolwachse, Ozokerit und Mikrowachse in Betracht.

Beispiele für inerte, für Nahrungsmittel geeignete anorganische Füllstoffe sind anorganische Materialien in Pulverform (anorganische Füllstoffe), z. B. Oxide wie Aluminiumoxid, Siliciumdioxid, Titandioxid, Silikate wie Natriumsilikat, Magnesiumsilikat, Talk, Calciumsilikat, Zinksilikat, Aluminiumsilikate wie Natriumaluminiumsilikat, Kaliumaluminiumsilikat, Calciumaluminiumsilikat, Bentonit, Kaolin und Natriumchlorid.

Zu den für Nahrungs- und Futtermittel geeigneten Hilfsstoffen zählen weiterhin Dispergiermittel, einschließlich lipophiler Dispergiermittel zur Dispergierung der Astaxanthinzusammensetzungen in lipophilen Trägern und Schutzkolloide zur Dispergierung der erfindungsgemäßen Astaxanthinzusammensetzungen in hydrophilen Trägern wie Wasser, weiterhin Antioxidantien (Oxidationsstabilisatoren) und für Nahrungsmittel zugelassene Farbstoffe.

Beispiele für Antioxidantien sind Tocopherole wie α-Tocopherol, α-Tocopherolpalmitat, α-Tocopherolacetat, tert.Butylhydroxytoluol, tert.-Butylhydroxyanisol, Ascorbinsäure, deren Salze und Ester wie z. B. Natriumascorbat, Calciumascorbat, Ascorbylphosphatester und Ascorbylpalmitat und Ethoxyquin. Die Antioxidantien sind, sofern erwünscht, in der erfindungsgemäßen Additiven typischerweise in Mengen von 0,01 bis 10 Gew.-%, bezogen auf das Gesamtgewicht des Additivs, enthalten.

Beispiele für Konservierungsmittel umfassen Benzoesäure und ihre Salze, insbesondere ihre Natrium-, Kalium- und Calciumsalze, 4-Hydroxybenzoesäure (PHB) und ihre Salze, insbesondere ihre Natrium-, Kalium- und Calciumsalze, die Salze der PHB-Alkylester wie das Natriumsalz des PHB-Methylesters, das Natriumsalz des PHB-Ethylesters und das Natriumsalz des PHB-Propylesters, Sorbinsäure und ihre Salze, insbesondere ihre Natrium-, Kalium- und Calciumsalze, Salze der Propionsäure wie insbesondere ihre Natrium-, Kalium- und Calciumsalze, Borsäure und Milchsäure und deren Salze. Konservierungsmittel sind, sofern erwünscht, in den Additiven typischerweise in Mengen von 0,001 bis 2 Gew.-%, bezogen auf das Gesamtgewicht der Additive, enthalten.

Typische lipophile Dispergiermittel sind Ascorbylpalmitat, Polyglycerin-Fettsäureester wie Polyglycerin-3-polyrizinoleat (PGPR90), Sorbitanfettsäureester, insbesondere Sorbitan-C₁₀-C₂₈-fettsäureester wie z. B. Mono- und Di-C₁₀-C₂₈-fettsäureester des Sorbitans wie Sorbitanmonolaurat, Sorbitanmonooleat und Sorbitanmonostearat (SPAN60), ethoxilierte Sorbitanfettsäureester wie PEG(20)-Sorbitolmonooleat, Monoester der Milchsäure mit gesättigten C₁₀-C₂₄-Fettsäuren, Zuckerester von gesättigten C₁₆-C₂₈-Fettsäuren, Propylenglycol-Fettsäureester sowie Phospholipide wie Lecithin. Lipophile Dispergiermittel sind, sofern erwünscht, in den Additiven typischerweise in Mengen von 0,01 bis 10 Gew.-%, bezogen auf das Gesamtgewicht der Additive, enthalten.

Schutzkolloide sind polymere Substanzen, die zur Dispergierung von wasserunlöslichen Feststoffen in wässrigen Zusammensetzungen geeignet sind. Geeignete Schutzkolloide, welche für Nahrungsmittel und Futtermittel zugelassen sind, umfassen beispielsweise
1) proteinische Schutzkolloiden,
2) Ligninsulfonsäuren, deren Salze, insbesondere deren Natriumsalze und Kaliumsalze,
3) Oligo- und Polysaccharide, einschließlich modifizierter Polysaccharide, wie beispielsweise Dextrine, Cellulose und Cellulosederivate wie Methylcellulose, Carboxymethylcellulose und deren Salze, Hydroxyethylcellulose, Hydroxypropylcellulose und Hydroxypropylmethylcellulose, Gummi-Arabicum, Pektine,
4) Polyvinylalkohol, einschließlich partiell verseiftem Polyvinylalkohol,
5) Polyvinylpyrrolidon und
Gemische der vorgenannten Schutzkolloide.

Bei den proteinischen Schutzkolloiden der Gruppe 1) handelt es sich um eine in Wasser lösliche oder quellbare polymere Substanz, die aus Aminosäuren aufgebaut ist und gegebenenfalls glykosidische Bestandteile aufweist. Das proteinische Schutzkolloid ist in der Regel pflanzlichen oder tierischen Ursprungs. Beispiele für proteinische Schutzkolloide der Gruppe 1) sind Caseine und Caseinate, einschließlich asi-, αₛ₂-, β- und κ-Kasein und deren Gemische, Prolamine, d. h. Speicherproteine aus Getreidesamen wie Gliadin, Secalin, Avenalin, Hordein, Zein, Oryzin und Kafirin, Molkeproteine wie beta-Lactoglobulin, alpha-Lactalbumin, Serumalbumin, Proteosepepton, Immunoglobuline und deren Gemische, Gelatine wie Rinder-, Schweine- und Fischgelatine, insbesondere sauer abgebaute Gelatine wie Gelatine A100 und A200, alkalisch abgebaute Gelatine wie Gelatine B100 und B200 sowie enzymatisch abgebaute Gelatinetypen wie die unter den Handelsbezeichnungen Gelita® Collagel A, Gelita® Sol DA, Gelita® Sol PA und Gelita® Sol LDA (DGF Stoess) erhältlichen Produkte, weiterhin Sojaprotein und teilhydrolysiertes Sojaprotein sowie Lupin-Protein. Unter Lupin-Protein versteht man aus Lupine gewonnene Pflanzenproteine. Gemäß einer bevorzugten Ausführungsform enthält die pulverförmige Zusammensetzung wenigstens ein proteinisches Schutzkolloid der Gruppe 1), gegebenenfalls in Kombination mit einem Oligo- oder Polysacharid der Gruppe 3). Das Molekulargewicht (Gewichtsmittel) des proteinischen Schutzkolloids liegt typischerweise im Bereich von 10 000 bis 250 000 Dalton, insbesondere im Bereich von 15 000 bis 200 000 Dalton und speziell im Bereich von 20 000 bis 180 000 Dalton. Bevorzugte Schutzkolloide sind Casein, Caseinate z. B. Na-Caseinat, Sojaprotein, teilhydrolysiertes Sojaprotein, Prolamin und Lupin-Protein.

Gemäß einer bevorzugten Gruppe von Ausführungsformen der Erfindung handelt es sich bei den Nahrungs- bzw. Futtermitteladditiven um eine Lösung oder Suspension der erfindungsgemäßen Astaxanthinzusammensetzung in einem für Nahrungs- oder Futtermittel geeigneten Öl oder Fett. Die Konzentration an Astaxanthin in derartigen Additivformulierungen liegt in der Regel im Bereich von 0,01 bis 30 Gew.-%, insbesondere im Bereich von 0,1 bis 10 Gew.-%, bezogen auf das Gesamtgewicht des Additivs. Im Falle einer Lösung liegt die Konzentration vorzugsweise im Bereich von 0,01 bis 3 Gew.-%, insbesondere im Bereich von 0,1 bis 2 Gew.-%. Im Falle einer Suspension liegt die Konzentration an Astaxanthin im Bereich von 1 bis 30 Gew.-%, insbesondere im Bereich von 2 bis 20 Gew.-%. Die Konzentration an Astaxanthin bezeichnete die Gesamtkonzentration an Astaxanthin in Form des Monoesters, des gegebenenfalls vorhandenen Diesters und des unveresterten Astaxanthins. Neben dem für Nahrungs- oder Futtermittel geeigneten Öl kann die Lösung die vorgenannten Additive wie lipophile Dispergiermittel und/oder Antioxidantien und/oder Farbstoffe, vorzugsweise in den vorgenannten Mengen, enthalten. Im Falle von Suspensionen der erfindungsgemäßen Astaxanthinzusammensetzung weisen in der Regel wenigstens 80 Gew.-%, insbesondere wenigstens 90 Gew.-% der Partikel des Astaxanthinzusammensetzung einen Partikeldurchmesser unterhalb 100 µm, insbesondere unterhalb 70 µm und speziell unterhalb 50 µm auf. Häufig weisen die Partikel der Astaxanthinzusammensetzung in der Suspension einen volumenmittleren Teilchendurchmesser (D_{4,3}-Wert) im Bereich von 0,2 bis 50 µm, insbesondere im Bereich von 0,3 bis 30 µm und speziell im Bereich von 0,5 bis 20 µm auf. Unter dem volumenmittleren Teilchendurchmesser versteht man den mittels Fraunhofer-Beugung an einer verdünnten 0,01 bis 0,1 gew.-%igen, speziell 0,05 gew.-%igen Suspension bestimmten volumenmittleren Teilchendurchmesser.

Bei den erfindungsgemäßen Additiven für Nahrungsmittel und Futtermittel kann es sich auch um Pulver handeln. Diese Pulver weisen in der Regel neben der erfindungsgemäßen Astaxanthinzusammensetzung auch andere Pulverbestandteile auf. Hierzu zählen insbesondere Rieselhilfsmittel und Füllstoffe.

Unter einem Rieselhilfsmittel, das auch als Antiagglomerationsmittel bezeichnet wird, versteht man eine feste, pulverförmige Substanz, die ein Verkleben der Pulverpartikel untereinander oder mit Behälterwänden verringert. Bei den Rieselhilfsmitteln handelt es sich typischerweise um inerte, für Nahrungsmittel geeignete feste anorganische Materialien in Pulverform, z. B. um Oxide wie Aluminiumoxid, Siliciumdioxid, insbesondere in Form von feinteiliger Kieselsäure wie pyrogene Kieselsäure oder Kieselgel (E 551), oder Titandioxid (E 171), Phosphate wie Tricalciumphosphat, Hydroxide wie Aluminiumhydroxid, Carbonate und Hydrogencarbonate wie Natriumcarbonat (E 500), Natriumhydrogencarbonat, Kaliumcarbonat (E 501), Magnesiumcarbonat (E 504) und Calciumcarbonat (E 170), oder Silikate wie Natriumsilikat (E 550), Magnesiumsilikat (E 553a), Talk (E 553b), Calciumsilikat (E 552), Zinksilikat (E 557), Aluminiumsilikate wie Natriumaluminiumsilikat (E 554), Kaliumaluminiumsilikat (E 555), Calciumaluminiumsilikat (E 556), Bentonit (E 558), Kaolin (E 559), Natriumchlorid, und/oder um inerte, für Nahrungsmittel geeignete feste organische Materialien in Pulverform, wie beispielsweise kristalline Mono-, Di- und höhere Polysacharide wie Lactose, Zucker (Saccharose), Cellulosepulver, Stearinsäure oder Stearate wie Magnesiumstearat (E 572), Ammoniumstearat (E 571) und Aluminiumstearat (E 573). Vorzugsweise weisen die Rieselhilfsmittel Teilchengrößen auf, die unterhalb der Teilchengröße der Pulverteilchen liegen. Vorzugweise weisen die Teilchen des Rieselhilfsmittels volumenmittlere Teilchendurchmesser im Bereich von 1/100 bis 1/2 des volumenmittleren Teilchendurchmessers der Pulverteilchen des Astaxanthin-Derivats auf. Der Anteil der Rieselhilfsmittel wird in der Regel 10 Gew.% nicht überschreiten und beträgt, sofern vorhanden, typischerweise 0,1 bis 10 Gew.-%, vorzugsweise 0,2 bis 5 Gew.-%, insbesondere 0,3 bis 3 Gew.-%, bezogen auf das Gesamtgewicht des pulverförmigen Additivs.

Die pulverförmigen Additivzusammensetzungen enthalten neben der Astaxanthinzusammensetzung ein oder mehrere weitere Pulverbestandteile. Hierzu zählen neben den vorgenannten Rieselhilfsmitteln insbesondere andere organische und anorganische Substanzen, welche für Nahrungsmittel geeignet sind und die vorzugsweise bei 50 °C, insbesondere bei 80 °C ein Feststoff sind. Hierzu zählen insbesondere die vorgenannten Füllstoffe, Schutzkolloide, Antioxidantion und Konservierungsmittel. In den erfindungsgemäßen pulverförmigen Zusammensetzungen weisen in der Regel wenigstens 90 Gew.-% der Pulverteilchen einen Teilchendurchmesser unterhalb 500 µm, insbesondere maximal 200 µm auf. Die Pulverteilchen weisen in der Regel eine mittlere Teilchengröße (Teilchendurchmesser, Volumenmittel, D_{4,3}-Wert) im Bereich von 0,5 bis 300 µm, insbesondere im Bereich von 0,5 bis 200 µm auf.

In einer Gruppe von Ausführungsformen erfindungsgemäßer pulverförmiger Nahrungs- und Futtermitteladditive umfasst die pulverförmige Formulierung wenigstens ein Schutzkolloid. Diese Zusammensetzungen sind in Wasser dispergierbar. Das Gewichtsverhältnis von Schutzkolloid zu den Astaxanthinbestandteilen der Astaxanthinzusammensetzung liegt typischerweise im Bereich von 50 : 1 bis 1 : 5, insbesondere im Bereich von 20 : 1 bis 1 : 2 und speziell im Bereich von 10 : 1 bis 1 : 1.

In bevorzugten Ausführungsformen pulverförmiger Nahrungs- und Futtermitteladditive umfasst die pulverförmige Formulierung wenigstens ein proteinisches Schutzkolloid. Gemäß einer ersten Gruppe ist das proteinische Schutzkolloid insbesondere ein Sojaprotein oder ein Gemisch aus Sojaprotein und einem weiteren proteinischen Schutzkolloid, z. B. ein Gemisch aus Sojaprotein und einem Prolamin, ein Gemisch aus Sojaprotein und einem Lupin-Protein, ein Gemisch aus Sojaprotein und einem Casein und/oder Caseinat, ein Gemisch aus Sojaprotein und Sojaproteinhydrolysat. Insbesondere bildet das Sojaprotein den Hauptbestandteil des proteinischen Schutzkolloids, der Anteil am Schutzkolloid beträgt wenigstens 50 Gew.-%, insbesondere wenigstens 80 Gew.-% oder wenigstens 90 Gew.-%. Gemäß einer zweiten Gruppe ist das proteinische Schutzkolloid insbesondre ein teilhydrolysiertes Sojaprotein oder ein Gemisch aus teilhydrolysiertem Sojaprotein und einem weiteren proteinischen Schutzkolloid, z. B. ein Gemisch aus teilhydrolysiertem Sojaprotein und einem Prolamin, ein Gemisch aus teilhydrolysiertem Sojaprotein und einem Lupin-Protein, ein Gemisch aus teilhydrolysiertem Sojaprotein und einem Casein und/oder Caseinat. Insbesondere bildet das teilhydrolysierte Sojaprotein den Hauptbestandteil des proteinischen Schutzkolloids, der Anteil am Schutzkolloid beträgt wenigstens 50 Gew.-%, insbesondere wenigstens 80 Gew.-% oder wenigstens 90 Gew.-%. Unter einem teilhydrolysierten Sojaprotein versteht man ein Sojaprotein, das einem partiellen enzymatischen Abbau mit einer Protease unterworfen wurde. Der Abbaugrad DH eines solchen teilhydrolysierten Sojaproteins beträgt in der Regel wenigstens 1 %, insbesondere wenigstens 5 %, z. B. im Bereich von 1 bis 20 % und insbesondere im Bereich von 5 bis 16 %. Unter dem Abbaugrad DH versteht man den Quotienten aus der Anzahl der hydrolysierten Peptidbindungen zur Gesamtzahl der Peptidbindungen im Ausgangsprotein. Der Abbaugrad kann gemäß der so genannten "pH-Stat-Methode" bestimmt werden, wie von C. F. Jacobsen et al. in "Methods of Biochemical Analysis", Vol. IV, S. 171-210, Interscience Publishers Inc., New York 1957, beschrieben. Als Sojaproteine für die Herstellung der erfindungsgemäßen Formulierungen, aber auch zur Herstellung der teilhydrolysierten Sojaproteine werden üblicherweise handelsübliche Sojaprotein-Isolate und - Konzentrate mit Proteingehalten von in der Regel 70 bis 90 Gew.-% eingesetzt, wobei die restlichen 10 bis 30 Gew.-% mehr oder weniger undefinierte andere Pflanzenbestandteile darstellen.

In bevorzugten Ausführungsformen pulverförmiger Nahrungs- und Futtermitteladditive umfasst die pulverförmige Formulierung wenigstens ein Schutzkolloid aus der Gruppe der Ligninsulfonate, insbesondere Na-Ligninsulfonat, gegebenenfalls in Kombination mit weiteren Schutzkolloiden, insbesondere solchen aus der Gruppe 3). Insbesondere bildet das Ligninsulfonat in dieser Ausführungsform den Hauptbestandteil des Schutzkolloids, der Anteil am Schutzkolloid beträgt wenigstens 50 Gew.-%, insbesondere wenigstens 80 Gew.-% oder wenigstens 90 Gew.-%.

Offenbart sind auch Nahrungsmittel, die eine erfindungsgemäße Astaxanthinzusammensetzung enthalten, wobei die Konzentration der Astaxanthinzusammensetzung dem für Nahrungsmittel zugelassenen Gehalt an Astaxanthin in dem Nahrungsmittel entspricht. Typischerweise liegt die Konzentration der erfindungsgemäßen Zusammensetzung, gerechnet als Astaxanthin, im Bereich von 1 bis 500 mg/kg des jeweiligen Nahrungsmittels.

Beispiele für Nahrungsmittel, die mit den erfindungsgemäßen Zusammensetzungen additiviert sind, umfassen fetthaltige Nahrung wie Wurst, Käse, Speiseöle, Aufbaunahrung, Proteinnahrung, Sportriegel (powerbar), Funktionsgetränke wie Sport- und Wellness-Getränke und hochkalorische Getränke, Astronautenkost, Sondennahrung und dergleichen.

Ferner offenbart sind Futtermittel, die eine erfindungsgemäße Astaxanthinzusammensetzung enthalten, wobei die Konzentration der Astaxanthinzusammensetzung dem für das jeweilige Futtermittel zugelassenen Gehalt an Astaxanthin in dem Futtermittel entspricht. Typischerweise liegt die Konzentration der erfindungsgemäßen Zusammensetzung, gerechnet als Astaxanthin, im Bereich von 1 bis 500 mg/kg des jeweiligen Futtermittels.

Typische Bestandteile in Futtermitteln sind Kohlehydrat-Quellen, insbesondere Getreidemehle wie Weizen- oder Maismehl, Sojabohnenmehl, aber auch Zucker und Zuckeralkohole, weiterhin proteinhaltige Bestandteile wie Sojakonzentrat, Fischmehl, Glutene wie Mais- oder Weizengluten, Öle und Fette, z. B. die zuvor genannten essbaren Öle, aber auch andere essbare Fette pflanzlichen oder tierischen Ursprungs, weiterhin Nutrazeutika wie freie Aminosäuren, deren Salze, Vitamine und Spurenelemente, sowie gegebenenfalls Verarbeitungshilfsmittel, z. B. Gleitmittel, Antiblockmittel, inerte Füllstoffe und dergleichen, und gegebenenfalls Konservierungsmittel. Typische Fischfutterzusammensetzungen enthalten z. B. Getreidemehl in einer Menge von beispielsweise 3 bis 20 Gew.-%, Gluten, z. B. in einer Menge von 1 bis 30 Gew.-%, ein oder mehrere Proteinquellen, z. B. Sojakonzentrat und/oder Fischmehl, z. B. in einer Gesamtmenge von 10 bis 50 Gew.-%, Fette und/oder Öle in einer Menge von beispielsweise 10 bis 50 Gew.-%, gegebenenfalls ein oder mehrere Vitamine in einer Gesamtmenge von beispielsweise 0,1 bis 2 Gew.-% und gegebenenfalls Aminosäuren in einer Menge von beispielsweise 0,1 bis 5 Gew.-%, jeweils bezogen auf die Gesamtmenge der Futtermittelbestandteile.

Die Herstellung der Nahrungsmittel bzw. der Futtermittel geling durch Additivierung des Nahrungsmittels mit einem erfindungsgemäßen Nahrungs- oder Futtermitteladditiv in der gewünschten Menge. Die Additivierung kann während der Konfektionierung des Nahrungs- oder Futtermittels oder durch Zugabe zu den Nahrungs- bzw. Futtermittelbestandteilen bei der Herstellung des Nahrungs- bzw. Futtermittels erfolgen.

Eine spezielle Ausführungsform dieser Futtermittel betrifft Futtermittelpellets, speziell Futtermittelpellets für Fischfutter, die mit der erfindungsgemäß erhältlichen Lösung des Astaxanthin-Derivats beladen sind. Derartige Pellets enthalten die erfindungsgemäße Astaxanthinzusammensetzung typischerweise in einer Menge von 10 bis 200 ppm, bezogen auf das Gesamtgewicht des Futtermittels und gerechnet als Astaxanthin. Die Herstellung solcher Pellets erfolgt in der Regel durch Besprühen konventioneller Pellets mit einer Lösung der erfindungsgemäßen Astaxanthinzusammensetzung, vorzugsweise unter vermindertem Druck, wobei das Besprühen kontinuierlich oder vorzugsweise diskontinuierlich erfolgen kann. Beispielsweise kann man die konventionellen Pellets in einem geeigneten Gefäß vorlegen, das Gefäß evakuieren und dann eine Lösung der Astaxanthinzusammensetzung in einem für Nahrungsmittel geeigneten Öl unter Durchmischen der Pellets aufsprühen und anschließend belüften. Auf diese Weise erreicht man ein gleichmäßiges Eindringen der öligen Lösung in die Pellets. Gegebenenfalls kann man erneut Vakuum anlegen und erneut die Lösung der Astaxanthinzusammensetzung in einem für Nahrungsmittel geeigneten Öl in der zuvor beschriebenen Weise aufsprühen. Auf diese Weise erhält man Pellets, die im Kern das Öl und die erfindungsgemäße Astaxanthinzusammensetzung enthalten.

Dier Erfindung betrifft auch Nahrungsergänzungsmittel. Diese Nahrungsergänzungsmittel enthalten die erfindungsgemäße Astaxanthinzusammensetzung in formulierter Form, d. h. sie umfassen neben der erfindungsgemäßen Astaxanthinzusammensetzung wenigstens einen für Nahrungsergänzungsmittel zugelassenen Hilfsstoff. Hierzu zählen vor allem die im Zusammenhang mit den Nahrungsmitteladditiven genannten Hilfsstoffe wie Träger, Dispergiermittel, Antioxidationsmittel, Konservierungsmittel und Farbstoffe sowie sonstige, für die Herstellung der gewünschten Darreichungsform notwendigen Hilfsstoffe.

Typische Darreichungsformen für Nahrungsergänzungsmittel sind Pulver, Puder, Granulate, Tabletten, insbesondere Filmtabletten, Pastillen, Sachets, Cachets, Dragees, Kapseln wie Hart- und Weichgelatinekapseln, Brausetabletten und Brausepulver.

Die Menge an Astaxanthinzusammensetzung in dem Nahrungsergänzungsmittel liegt typischerweise im Bereich von 0,5 bis 100 mg, insbesondere 1 bis 50 mg pro Dosiseinheit, gerechnet als Astaxanthin.

Gegenstand der Erfindung sind die hier beschriebenen Astaxanthinzusammensetzungen zur therapeutischen Verwendung als Medikament sowie als Inhaltsstoff für eine medizinische Zubereitung (= Pharmazeutisches Mittel). Geeignete Indikationen, in denen die erfindungsgemäßen Zusammensetzungen eingesetzt werden können, sind die in Anspruch 11 genannten Indikationen.

Dabei wird dem zu behandelnden Individuum, vorzugsweise einem Säuger, insbesondere einem Menschen, Nutz- oder Haustier, eine wirksame Menge erfindungsgemäßen Astaxanthinzusammensetzung, in der Regel der pharmazeutischen und tierarzneilichen Praxis entsprechend formuliert, verabreicht. Ob eine solche Behandlung angezeigt ist und in welcher Form sie zu erfolgen hat, hängt vom Einzelfall ab und unterliegt einer medizinischen Beurteilung (Diagnose), die vorhandene Anzeichen, Symptome und/oder Fehlfunktionen, Risiken, bestimmte Anzeichen, Symptome und/oder Fehlfunktionen zu entwickeln, und weitere Faktoren miteinbezieht.

Die Behandlung erfolgt in der Regel durch einmalige oder mehrmalige tägliche Verabreichung, gegebenenfalls zusammen oder im Wechsel mit anderen Wirkstoffen oder wirkstoffhaltigen Präparaten, so dass einem zu behandelnden Individuum eine Tagesdosis von vorzugsweise etwa 0,1 bis 50 mg/kg Körpergewicht, insbesondere 0,5 bis 10 mg/kg Körpergewicht, oder 1 bis 1000 mg, insbesondere 2 bis 200 mg, jeweils gerechnet als Astaxanthin, vorzugsweise mittels oraler Gabe zugeführt wird.

Weiterhin wird auch die Herstellung medizinischer Zubereitungen, im Folgenden auch als pharmazeutische Mittel bezeichnet, zur Behandlung eines Individuums, vorzugsweise eines Säugers, insbesondere eines Menschen, Nutz- oder Haustieres offenbart. So werden die erfindungsgemäßen Astaxanthinzusammensetzungen gewöhnlich in Form von medizinischen Zubereitungen verabreicht, die einen pharmazeutisch verträglichen Hilfsstoff, d. h. einen Exzipienten, die erfindungsgemäße Astaxanthinzusammensetzung und gegebenenfalls einen oder mehrere weitere Wirkstoffen umfassen. Diese Zubereitungen werden vorzugsweise auf oralem Wege verabreicht.

Beispiele geeigneter medizinischer Zubereitungen umfassen feste Arzneiformen, wie Pulver, Puder, Granulate, Tabletten, insbesondere Filmtabletten, Pastillen, Sachets, Cachets, Dragees, Kapseln wie Hart- und Weichgelatinekapseln, Brausetabletten, Brausepulver, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Augen- und Ohrentropfen. Ferner können auch Liposomen oder Mikrokapseln zur Anwendung kommen.

Bei der Herstellung der medizinischen Zubereitungen werden erfindungsgemäße Astaxanthinzusammensetzungen gewöhnlich mit einem Exzipienten vermischt oder verdünnt. Exzipienten können feste, halbfeste oder flüssige Materialien sein, die als Vehikel, Träger oder Medium für den Wirkstoff dienen. Die erfindungsgemäßen Zusammensetzungen enthalten typischerweise die Astaxanthinzusammensetzung in einer Menge die der Tagesdosis oder einem Bruchteil der Tagesdosis, z. B. der halben, einem Drittel oder einem Viertel der Tagesdosis entspricht. Die Menge an Astaxanthinzusammensetzung in der medizinischen Zubereitung liegt typischerweise im Bereich von 0,5 bis 200 mg, insbesondere 1 bis 100 mg pro Dosiseinheit, gerechnet als Astaxanthin.

Geeignete Exzipienten sind in den einschlägigen Arzneimonographien gelistet. Ferner können die Formulierungen pharmazeutisch akzeptable Träger oder übliche Hilfsstoffe, wie Gleitmittel; Netzmittel; emulgierende und suspendierende Mittel; konservierende Mittel; Antioxidantien; Antireizstoffe; Chelatbildner; Dragierhilfsmittel; Emulsionsstabilisatoren; Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist.

Für die folgenden Untersuchungen wurden die folgenden Astaxanthinzusammensetzungen eingesetzt. Die Abkürzung FI.-% steht für die mittels HPLC bestimmten Flächenprozente:
Astaxanthinmonooleat mit folgenden Eigenschaften:
   Gehalt an Astaxanthinmonooleat > 91 Gew.-%;
   E/Z-Isomerenverteilung im Astaxanthingerüst > 45 FI.-% all-E-Isomer;
   Enantiomerenreinheit: > 95 %ee 3S,3'S-Enantiomer;
   Anteil trans-Fettsäureester > 40 Gew.-%.
Astaxanthindioleat mit folgenden Eigenschaften:
   Gehalt an Astaxanthindioleat > 93 Gew.-%;
   E/Z-Verhältnis im Astaxanthingerüst > 60 FI.-% all-E-Isomer;
   Enantiomerenreinheit: > 95 %ee 3S,3'S-Enantiomer;
   Anteil trans-Fettsäureester 60 Gew.-%.
Astaxanthin-Naturprodukt aus
   Gemisch von Mono- und Diestern des Astaxanthins (überwiegend 3S,3'S-Enantiomer) mit C₁₆-C₂₂-Fettsäuren aus Blutregenalge (*Haematococcus pluvialis*) mit folgender Zusammensetzung (bestimmt mittels HPLC-MS, Angaben in Flächen-%)
   1,6 % Astaxanthin-C18:4-monoester
   9,7 % Astaxanthin-C18:3-monoester
   15,0 % Astaxanthin-C18:2-monoester
   28,7 % Astaxanthin-C18:1-monoester
   5,3 % Astaxanthin-C18:0-monoester

Die übrigen 39,7 % verteilt sich auf 77 nicht identifizierte Peaks mit jeweils < 2 FI.-%, bei denen es sich aber vor allem um Mono- und Diester des Astaxanthins handelt.

Herstellungsbeispiel 1: Herstellung von Astaxanthinmonopalmitat
3 g (11,7 mmol) Palmitinsäure wurden in 47,37 ml (53 g, 740 mmol) Dichlormethan vorgelegt. Bei Raumtemperatur wurden 2,85 g (17,55 mmol) 1,1'-Carbonyldiimidazol (CDI) in drei Portionen im Abstand von jeweils 5 Minuten zugegeben. Es wurde über Nacht nachgerührt und am nächsten Tag 3,49 g (5,85 mmol) Astaxanthin (3S,3'S-Enantiomer, ee > 95 %) zugegeben. Nach 6 Stunden gab man 133,8 µl Essigsäure zum Reaktionsgemisch und rührte über Nacht bei Raumtemperatur nach. Nach 20 Stunden wurde eine Probe dünnschichtchromatographisch untersucht. (Kieselgel, Laufmittel Cyclohexan/Essigsäureethylester = 1:2). Das Dünnschichtchromatogramm zeigte, dass in Teil des eingesetzten Astaxanthins zum Astaxanthinmonopalmitat umgesetzt worden war. Astaxanthindipalmitat ließ sich nur in sehr geringen Mengen nachweisen.

Eine Abtrennung des Astaxanthinmonopalmitats von den Reagenzien und dem nicht umgesetzten Astaxanthins gelingt durch Chromatographie des Reaktionsgemischs an Kieselgel mit Cyclohexan/Essigsäureethylester im Volumenverhältnis 1:2. Auf diese Weise kann man Astaxanthinmonopalmitat mit einer Reinheit > 80 Gew.-%, insbesondere > 90 Gew.-% erhalten. In analoger Weise gelingt die Gewinnung von Astaxanthinmonooleats mit einer Reinheit > 80 Gew.-%, insbesondere > 90 Gew.-%.

### Untersuchungen zur Bioverfügbarkeit

Die Untersuchung zur Bioverfügbarkeit erfolgte durch Bestimmung des Blutplasmaspiegels von Astaxanthin in Göttinger Minischweinen. Als Maß für die Bioverfügbarkeit wurde die Integrale Plasmakonzentration im Blutserum über 96 h (AUC) ermittelt.

Alle Astaxanthin-derivate wurden als Lösungen in Olivenöl verabreicht:
- Formulierung 1:: Astaxanthinmonooleat, 4,2 Gew.-% (berechnet als freies Astaxanthin) in Olivenöl
- Formulierung 2:: Astaxanthindioleat, 3,1 Gew.-% (berechnet als freies Astaxanthin) in Olivenöl
- Formulierung 3:: Astaxanthinnaturprodukt, 2,4 Gew.-% (berechnet als freies Astaxanthin) in Olivenöl

Formulierungen 2 und 3 stellen Vergleichsbeispiele dar. Die Untersuchung erfolgte an drei 4-5 Monate alten, männlichen Göttinger Minischweinen mit einem Körpergewicht von 9,5 - 10,5 kg. Allen Minischweinen wurde zur Blutentnahme ein Katheter in die Vena femoralis implantiert. Zwei der Minischweinen (Schwein 2 und Schwein 3) wurde zudem ein Katheter in die Jugularvene implantiert. Während des Untersuchungszeitraums wurden die Katheter täglich mit physiologischer Kochsalzlösung gespült.

Während der Nichtbehandlungszeiten wurden die Minischweine mit einer kommerziellen Diät für Minischweine gefüttert (Firma SDS, Essex, UK). Die Diät wurde werktags am Morgen und am Nachmittag und am Wochenende am frühen Morgen und kurz vor Mittag gefüttert.

Am Tag der Behandlung erfolgte die Fütterung mit einer mit 20 % Maisöl angereicherten fettreichen Diät. Hierzu wurden 224 g der kommerziellen Minischwein-Diät mit 54 g Maisöl vermengt. Schwein 1 erhielt von der fettreichen Diät 140 g unmittelbar vor der 1. und 2. Behandlung und 140 g unmittelbar nach der 1. und 2. Behandlung sowie 140 g 2 h vor der 3. Behandlung und 140 g 1 h nach der 3. Behandlung. Die Schweine 2 und 3 erhielten von der fettreichen Diät je 170 g unmittelbar vor der 1. und 2. Behandlung und 110 g unmittelbar nach der 1. und 2. Behandlung sowie 170 g 2 h vor der 3. Behandlung und 110 g 1 h nach der dritten Behandlung.

Zwischen den Behandlungen lag jeweils ein Zeitraum von 1 Woche. Das Behandlungsschema war wie in Tabelle 1 dargestellt:

**Tabelle 1: Behandlungsschema**

| | 1. Behandlung | 2. Behandlung | 3. Behandlung |
|---|---|---|---|
| Schwein 1 | Formulierung 3 | Formulierung 2 | Formulierung 1 |
| Schwein 2 | Formulierung 1 | Formulierung 3 | Formulierung 2 |
| Schwein 3 | Formulierung 2 | Formulierung 1 | Formulierung 3 |

Zur Behandlung wurde dem jeweiligen Schwein die Formulierung mittels einer Magensonde als Einzeldosis verabreicht. Die Einzeldosis lag bei 50 mg/kg Astaxanthin. Unmittelbar vor der Verabreichung sowie 2 h, 6 h, 7 h, 8 h, 9 h, 10 h, 11 h, 12 h, 1 h, 24 h, 32 h, 48 h, 56 h, 72 h und 96 h nach der Verabreichung wurde den Schweinen etwa 2 ml Blut über die Vena femoralis entnommen. Die Proben wurden auf Eis gelagert und innerhalb von 30 min. nach Entnahme 10 min. bei 1500 g und 4-8°C zentrifugiert. Das Plasma wurde in sterile, verschließbare Polypropylen-Behälter (Micronic, Lelystadt) überführt und bei Temperaturen unterhalb -75°C bis zur Analyse gelagert. Die Bestimmung des Astaxanthinspiegels im Blutplasma erfolgte mittels HPLC durch die Fa. WIL Research, Den Bosch NL. Die Ergebnisse sind in der nachfolgenden Tabelle 2 dargestellt. Angegeben sind die Mittelwerte für die drei Testschweine.

**Tabelle 2:**

| | | | |
|---|---|---|---|
| tₗₐₛₜ [h] | 48-56 | 48-56 | 24-23 |
| tₘₐₓ [h] | 6-12 | 8-11 | 6-9 |
| Cmax [ng/mL] | 61,8 | 89,9 | 5,54 |
| Cₗₐₛₜ [ng/mL] | 2,83 | 6,38 | 1,71 |
| AUCₗₐₛₜ [h*ng/mL] | 941 | 1560 | 65,6 |
| AUC_{∞} [h∗ng/mL] | 996 | 1670 | 118 |
| t_{1/2} [h] | 10,3 | 9,95 | 18,9 |

| | | | |
|---|---|---|---|
| tₗₐₛₜ = Zeitintervall bis zum Unterschreiten der Nachweisgrenze tₘₐₓ = Zeitintervall des maximalen Serumspiegels Cₘₐₓ = Maximaler Serumspiegel Cₗₐₛₜ = Konzentration vor Unterschreiten der Nachweisgrenze AUClast = Konzentrationsintegral bis zum Zeitpunkt vor Unterschreiten der Nachweisgrenze AUC_{∞} = extrapoliertes Konzentrationsintegral t_{1/2} = Halbwertszeit | | | |

## Patentansprüche

1. Verwendung von Astaxanthinzusammensetzungen in Nahrungsmitteln, Nahrungsergänzungsmitteln oder Futtermitteln, wobei die Astaxanthinzusammensetzung wenigstens 90 Gew.-%, bezogen auf das Gesamtgewicht an Astaxanthin und Astaxanthinderivaten in der Zusammensetzung, Monoester des Astaxanthins mit einer C18-1-Fettsäure enthält.

2. Verwendung nach Anspruch 1, worin weniger als 60 Gew.-% der im Monoester des Astaxanthins enthaltenen ungesättigten Fettsäuren als trans-Fettsäuren vorliegen.

3. Verwendung nach einem der vorhergehenden Ansprüche, worin wenigstens 40 Gew.-% des Monoesters des Astaxanthins in Form von Monoestern des all-trans-Astaxanthins vorliegen.

4. Verwendung nach einem der vorhergehenden Ansprüche, worin wenigstens 90 Gew.-% des Monoesters des Astaxanthins in Form von Monoestern eines Astaxanthins vorliegen, das in den Asymmetriezentren in Position 3 und 3', entweder jeweils (S)- oder jeweils (R)-konfiguriert ist.

5. Zusammensetzung in Form eines Nahrungsmitteladditivs, Futtermitteladditivs oder Nahrungsergänzungsmittels, enthaltend wenigstens 90 Gew.-%, bezogen auf das Gesamtgewicht an Astaxanthin und Astaxanthinderivaten in der Zusammensetzung, Monoester des Astaxanthins mit einer C18-1-Fettsäure, und einen für Nahrungsmittel, Futtermittel oder Nahrungsergänzungsmittel geeigneten Hilfsstoff.

6. Zusammensetzung nach Anspruch 5, ausgewählt unter Lösungen und Suspension der Astaxanthinzusammensetzung und einem für Nahrungs- oder Futtermittel geeigneten Öl oder Fett.

7. Zusammensetzung nach Anspruch 5 in Form eines Pulvers, umfassend eine Astaxanthinzusammensetzung und ein Schutzkolloid.

8. Zusammensetzung nach Anspruch 5 in Form eines Nahrungsergänzungsmittels zur oralen Verabreichung.

9. Verfahren zur Herstellung eines Nahrungsmittels oder Futtermittels, umfassend die Additivierung eines konventionellen Nahrungsmittels bzw. Futtermittels mit einer Astaxanthinzusammensetzung gemäß Definition in einem der Ansprüche 1 bis 4.

10. Astaxanthinzusammensetzung gemäß Definition in einem der Ansprüche 1 bis 4 zur therapeutischen Verwendung als Medikament sowie als Inhaltsstoff für eine medizinische Zubereitung.

11. Astaxanthinzusammensetzung nach Anspruch 10 zur Behandlung oder Vorbeugung von Gelenkerkrankungen, Augenerkrankungen, Hauterkrankungen, Herzerkrankungen, Erkrankungen der Blutgefäße, entzündlichen Erkrankungen, Tumorerkrankungen, bakteriellen Infektionen, Diabetes Typ2 und damit verbundener Folgeerkrankungen, degenerativen Nervenstörungen und zur Stärkung des Immunsystems.

## Claims

1. The use of astaxanthin compositions in foodstuffs, food supplements or feedstuffs, where the astaxanthin composition comprises at least 90% by weight, based on the total weight of astaxanthin and astaxanthin derivatives in the composition, of monoesters of astaxanthin with a C18-1 fatty acid.

2. The use according to claim 1, wherein less than 60% by weight of the unsaturated fatty acids which are present in the astaxanthin monoester are present as trans-fatty acids.

3. The use according to any of the preceding claims, wherein at least 40% by weight of the astaxanthin monoester are present in the form of monoesters of all-trans astaxanthin.

4. The use according to any of the preceding claims, wherein at least 90% by weight of the astaxanthin monoester are present in the form of monoesters of an astaxanthin which either has the (S) or the (R) configuration in the asymmetric centers in positions 3 and 3', respectively.

5. A composition in the form of a food additive, feed additive or food supplement, comprising at least 90% by weight, based on the total weight of astaxanthin and astaxanthin derivatives in the composition, of monoesters of astaxanthin with a C18-1 fatty acid and an adjuvant which is suitable for foodstuffs, feedstuffs or food supplements.

6. The composition according to claim 5, selected among solutions and suspensions of the astaxanthin composition and an oil or fat which is suitable for foodstuffs or feedstuffs.

7. The composition according to claim 5 in the form of a powder, comprising an astaxanthin composition and a protective colloid.

8. The composition according to claim 5 in the form of a food supplement for oral administration.

9. A process for the preparation of a foodstuff or feedstuff, comprising the addition of an astaxanthin composition according to the definition in any of claims 1 to 4 to a conventional foodstuff or feedstuff.

10. The astaxanthin composition according to the definition in any of claims 1 to 4 for the therapeutic use as medicament and as constituent for a medicinal preparation.

11. The astaxanthin composition according to claim 10 for the treatment or prevention of joint diseases, eye diseases, skin diseases, heart diseases, diseases of the blood vessels, inflammatory diseases, tumor diseases, bacterial infections, type-2 diabetes and associated sequelae, degenerative nerve disorders and for strengthening the immune system.

## Revendications

1. Utilisation de compositions d'astaxanthine dans des produits alimentaires, des compléments alimentaires ou des fourrages, la composition d'astaxanthine contenant au moins 90 % en poids, par rapport au poids total d'astaxanthine et de dérivés d'astaxanthine dans la composition, de monoesters d'astaxanthine avec un acide gras en C18-1.

2. Utilisation selon la revendication 1, dans laquelle moins de 60 % en poids des acides gras insaturés contenus dans le monoester d'astaxanthine se présentent sous la forme d'acides gras trans.

3. Utilisation selon l'une quelconque des revendications précédentes, dans laquelle au moins 40 % en poids du monoester d'astaxanthine se présente sous la forme de monoesters d'astaxanthine all-trans.

4. Utilisation selon l'une quelconque des revendications précédentes, dans laquelle au moins 90 % en poids du monoester d'astaxanthine se présente sous la forme de monoesters d'une astaxanthine qui est configurée au niveau des centres d'asymétrie en position 3 et 3' à chaque fois sous forme (S) ou à chaque fois sous forme (R) .

5. Composition sous la forme d'un additif pour produit alimentaire, d'un additif pour fourrage ou d'un complément alimentaire contenant au moins 90 % en poids, par rapport au poids total d'astaxanthine et de dérivés d'astaxanthine dans la composition, de monoesters d'astaxanthine avec un acide gras en C18-1, et un adjuvant approprié pour les produits alimentaires, les fourrages ou les compléments alimentaires.

6. Composition selon la revendication 5, choisie parmi les solutions et les suspensions de la composition d'astaxanthine et d'une huile ou d'une matière grasse appropriée pour les produits alimentaires ou les fourrages.

7. Composition selon la revendication 5, sous la forme d'une poudre, comprenant une composition d'astaxanthine et un colloïde protecteur.

8. Composition selon la revendication 5 sous la forme d'un complément alimentaire pour administration orale.

9. Procédé de fabrication d'un produit alimentaire ou d'un fourrage, comprenant la supplémentation d'un produit alimentaire ou d'un fourrage classique avec une composition d'astaxanthine selon la définition dans l'une quelconque des revendications 1 à 4.

10. Composition d'astaxanthine selon la définition dans l'une quelconque des revendications 1 à 4, destinée à une utilisation thérapeutique en tant que médicament, ainsi qu'en tant que composant d'une préparation médicale.

11. Composition d'astaxanthine selon la revendication 10, destinée au traitement ou à la prévention de maladies articulaires, de maladies oculaires, de maladies cutanées, de maladies cardiaques, de maladies des vaisseaux sanguins, de maladies inflammatoires, de maladies tumorales, d'infections bactériennes, du diabète de type 2 et ses complications, de troubles nerveux dégénératifs et pour le renforcement du système immunitaire.
